# EUROPÄISCHE PATENTSCHRIFT

(11) **EP 0 977 614 B1**
(45) Veröffentlichungstag und Bekanntmachung des Hinweises auf die Patenterteilung: **29.06.2005**
(21) Anmeldenummer: 98921456.4
(22) Anmeldetag: 11.04.1998
(51) Int. Cl.: A61N 1/30, A61M 31/00

(54) **VORRICHTUNG ZUM BEHANDELN VON MALIGNEN, TUMORÖSEN GEWEBEBEREICHEN**
DEVICE FOR TREATING MALIGNANT, TUMOROUS AREAS OF TISSUE
DISPOSITIF POUR LE TRAITEMENT DE ZONES TISSULAIRES TUMORALES MALIGNES

(30) Priorität: 23.04.1997 DE 19717023
(43) Veröffentlichungstag der Anmeldung: 09.02.2000
(73) Patentinhaber: Micronas GmbH, 79108 Freiburg (DE)
(72) Erfinder: SIEBEN, Ulrich, D-79276 Reute (DE); WOLF, Bernhard, D-79252 Stegen (DE)
(74) Vertreter: Patentanwälte Westphal, Mussgnug & Partner
(86) Internationale Anmeldenummer: PCT/EP1998/002133
(87) Internationale Veröffentlichungsnummer: WO 1998/047561

(56) Entgegenhaltungen:
- EP-A- 0 510 857
- WO-A-94/01165
- WO-A-94/05361
- US-A- 4 425 117
- US-A- 5 301 688
- US-A- 5 505 700

## Beschreibung

Die Erfindung bezieht sich auf eine Vorrichtung zum Behandeln von malignen, tumorösen Gewebebereichen, mit wenigstens einem Meßsensor zur Bestimmung chemischer oder physikalischer Signalmuster in der unmittelbaren Umgebung des tumorösen Gewebebereiches, mit einer Steuereinrichtung sowie mit zumindest einer Behandlungseinrichtung, die wenigstens einen WirkstoffVorratsbehälter, eine Dosiereinrichtung mit Dosiersteuerung, eine Wirkstoffabgabeeinrichtung zur chemischen Beeinflussung und optional Elektroden zur physikalischen Beeinflussung des zu behandelnden, tumorösen Gewebebereiches aufweist und wobei der oder die Sensoren, die Wirkstoffabgabeeinrichtung sowie die Behandlungs-Elektroden mit der Steuereinrichtung für eine in Abhängigkeit von den Meßwerten der Tumorzellen gesteuerte, physikalische und/oder chemische Behandlung des tumorösen Gewebebereiches mit laufender Dosiernachführung mit dem zu applizierenden Wirkstoff verbunden sind.
Bei der Krebstherapie ist es beispielsweise bereits bekannt, Chemotherapeutika einzusetzen, die die pathogenen Organteile, möglichst aber nicht den übrigen Organismus schädigen sollen. Die systemische und regionale Dosierung der Chemotherapeutika ist jedoch problematisch, da einerseits durch eine entsprechende wirkstoffkonzentration eine hohe Wirksamkeit gegenüber zum Beispiel einem Tumor angestrebt wird, andererseits jedoch durch unspezifische Aufnahme aber dann die Gefahr der Schädigung des gesunden Gewebes besteht.

Aus der Druckschrift "BRINTON, J., B.: System to detect and treat cancer, using microwaves for both tasks. In: Elektronics, 1979, NR. 26, Seite 42 " ist ein System zum Detektieren und Behandeln von Krebs bekannt. Dabei werden Tumore mit Hilfe von Mikrowellen lokalisiert und dann zerstört. Insbesondere wird mit Hilfe eines Radiometers die geringfügig erhöhte Temperatur im Bereich eines Tumors zum Detektieren ausgenützt und dadurch der Tumor erkannt. Es handelt sich hierbei um eine einmalige, kurzzeitige Überhitzung und Zerstörung des Tumors. Eine solche Behandlung kann zwar bei sehr kleinen Tumoren vorgenommen werden, bei größeren Tumorbereichen ist diese Methode jedoch nicht einsetzbar.
Aus der DE 196 01 487 ist eine Vorrichtung zum Einsatz bei direkt oder operativ zugänglichen Bereichen bekannt, die die Merkmale des Oberbegriffs des Anspruchs 1 aufweist. Für Bereiche, die nicht operativ eröffnet werden sollen, ist eine solche Vorrichtung jedoch nicht einsetzbar.

Aufgabe der vorliegenden Erfindung ist es, eine Vorrichtung zu schaffen, mit der eine Target-orientierte Medikamentenabgabe möglich ist, bei der die Belastung und Schädigung der nicht betroffenen Bereiche des Körpers zumindest weitgehend reduziert ist.

Zur Lösung dieser Aufgabe wird vorgeschlagen, daß die Vorrichtung kapselförmig oder drageeförmig zum Schlucken ausgebildet ist und eine Umhüllung aufweist, die zumindest den oder die Sensoren abdeckt, daß die Umhüllung unter dem Einfluß von Körperflüssigkeit zumindest bereichsweise auflösbar (entfernbar) ist, daß ein Sensor zum Erfassen von Auslöse-Parametern und die Steuereinrichtung mittels eines Schwellwertschalters zur Aktivierung der Wirkstoffabgabeeinrichtung und/oder der Elektroden bei Überschreitung eines vorgegebenen oder vorgebbaren Sollwertes ausgebildet sind und daß zur Wirkstoffabgabe innerhalb des Wirkstoff-Vorratsbehälters wenigstens ein Heiz-Widerstand zum Erwärmen und zur Abgabe von Wirkstoff vorgesehen ist.

Aus der WO-A-94/01165 ist eine kapselförmige Vorrichtung zur Medikamentenabgabe bekannt, die mit Hilfe eines vorprogrammierten Mikroprozessors eine gesteuerte Abgabe eines Medikamentes ermöglicht. Der Beginn der Medikamentenabgabe wird durch den Kontakt der Kapsel zum Beispiel mit der Magensäure ausgelöst, jedoch erfolgt die Medikamentenabgabe dann nach einem vor dem Schlucken der Kapsel vorgegebenen Schema. Eine Änderung der Dosierung in Anpassung an die tatsächlichen Gegebenheiten ist dann nicht mehr möglich.

Mit der erfindungsgemäßen Vorrichtung kann eine Target-orientierte Medikamentenabgabe nach einer oralen Aufnahme erfolgen. Dabei wird, nachdem sich die Vorrichtung im Bereich eines zu behandelnden Tumors oder dergleichen befindet, zur, chemischen Beeinflussung ein medizinischer Wirkstoff appliziert und gleichzeitig erfolgt dort auch in unmittelbarer Umgebung des Behandlungsortes mit Hilfe des oder der Sensoren während der Behandlung eine laufende Überwachung. Aufgrund der Meßwerte kann die Dosierung entsprechend den Sollwertvorgaben von der Steuereinrichtung angepaßt werden. Es ist somit ein selbständig arbeitender Regelkreis gebildet, durch den eine laufende Dosierungsnachführung mit dem zu applizierten Wirkstoff vorgenommen werden kann.
Bei diesem Regelprozeß wird somit die Wirkstoffabgabe bei einer chemischen Behandlung und/oder die Intensität bei der physikalischen Behandlung in Abhängigkeit von einem Meßwert gesteuert. Es wird somit nicht nur eine Behandlung ausgelöst, sondern die Behandlung erfolgt durch Messung von Auslöse-Parametern, beispielsweise des pH-Wertes. Dies bedeutet auch, daß bei Unterschreiten eines Schwellwertes die Behandlung aufhört.
Es ist somit eine Target-orientierte Medikamtenabgabe und somit eine Behandlung in selektierter Weise möglich, denn nach dem Schlucken der Kapsel erfolgt zunächst das Entfernen der SchutzUmhüllung, so daß dann der oder die Sensoren freiliegen und in der Lage sind, vorgebbare Auslöse-Parameter zu erfassen. Gelangt die Kapsel in diesem aktivierten Zustand in den Bereich von malignem, tumorösen Gewebe, so wird dies anhand z.B. des pH-Wertes erkannt und je nach Abweichung des pH-Wertes vom normalen pH-Wert in dieser Körperumgebung, wird eine chemische und/oder physikalische Behandlung geregelt durchgeführt. Beim Weitertransport der Kapsel und dem Austreten aus dem Bereich des zu behandelnden, tumorösen Gewebes, wird die Behandlung reduziert und schließlich eingestellt.
Die Wirkstoffabgabe erfolgt durch Erwärmen des Wirkstoffs, wobei der Wirkstoff-Vorratsbehälter vorzugsweise wenigstens eine Kapillare zur Wirkstoffabgabe aufweist. Durch den thermischen Einfluß des Heizwiderstandes dehnt sich die Flüssigkeit aus und wird aus dem Vorratsbehälter in Richtung Auflage- und Kontaktfläche abgegeben. Die Abgabe der Wirksubstanz über die Auflage-und Kontaktfläche kann auch sowohl iontophoretisch als auch thermisch über eine entsprechende thermische Mikropumpe oder ein thermisches Ventil erfolgen.
Mit der erfindungsgemäßen Vorrichtung kann auch eine Target-orientierte Medikamentenabgabe durch orale Aufnahme bei Magen- und Darmerkrankungen, beispielsweise durch eine gezielte Alkaliabgabe oder die Abgabe eines anderer Wirkstoffes vorgenommen werden.
Auslöse-Parameter für den Beginn der Behandlung kann dabei der pH-Wert sein, der zum Beispiel laufend gemessen wird und bei Abweichung von einem vorgegebenen Wert die Behandlung insbesondere durch Wirkstoffabgabe auslöst. Außer der beispielsweise erwähnten pH-Wert-Messung kann auch eine Laktatmessung, eine Kalziummessung, eine Impedanzmessung und dergleichen vorgenommen werden.

Die Vorrichtung kann innerhalb des Verdauungstraktes ohne operativen Eingriff an vorbestimmbaren Stellen plaziert werden und es kann dort eine gezielte Behandlung vorgenommen werden oder es können über den Verdauungstrakt gezielt Medikamente abgegeben werden.

Durch die Umhüllung ist die Kapsel oder das Dragee vor dem Schlucken inaktiv und wird erst dann aktiv, wenn die Umhüllung aufgelöst ist. Die Umhüllung bildet auch praktisch eine-Schutzumhüllung, die bei längerer Aufbewahrung vor dem Behandlungseinsatz vorteilhaft ist.
Für eine exakte Positionierung beziehungsweise Aktivierung der Vorrichtung in einem vorgesehenen Behandlungsbereich innerhalb des Verdauungstraktes, ist die Umhüllung insbesondere zeit-und/oder stoffabhängig von der Körperflüssigkeit auflösbar. Durch unterschiedliche Umhüllungsstoffe und auch durch deren Wandungsdicke kann die Position der Vorrichtung innerhalb des Verdauungstraktes bei deren Aktivierung und auch der Aktivierungs-Zeitpunkt genügend genau vorbestimmt werden. Im Verlauf des Verdauungstraktes wirken unterschiedliche Verdauungssäfte auf die Umhüllung ein, so daß durch gezielte Auswahl des Umhüllungsstoffes festgelegt werden kann, unter dem Einfluß welches Verdauungsaftes die Aktivierung der Vorrichtung erfolgen soll.
Auch die Dauer des Aktivierungsvorganges, das heißt die Zeit zum Auf- oder Ablösen der Umhüllung, kann durch den Umhüllungsstoff und dessen Dicke vorbestimmt werden.
Es besteht auch die Möglichkeit, daß die von der Umhüllung abgedeckten Elektroden, die Abgabestelle und dergleichen nacheinander entsprechend dem vorgesehenen Behandlungsablauf freigelegt werden.

Eine Ausgestaltung der Erfindung sieht vor, daß zumindest zur Vergrößerung der Verweildauer der Kapsel oder des Dragees am Behandlungsort, diese eine Umhüllung mit einem Füllmaterial oder Aufschäumsystem insbesondere auf enzymatischer Basis aufweist, die für eine immunbiochemische und/oder immunenzymatische Reaktion bei Erreichen eines Entzündungs- oder tumorösen Ortes ausgebildet ist.
Durch die entsprechende Reaktion wird das Füllmaterial der Kapsel zum Andocken an die betreffende Stelle veranlaßt und gegebenenfalls durch eine chemische Reaktion ein Aufschäumen oder Aufquellen und damit eine Volumenvergrößerung bewirkt. Dadurch kann die Verweildauer am Behandlungsort zumindest verlängert werden, da ein "Verankern" am Behandlungsort eintritt. Das Aufschäum- oder Aufquellmaterial hat bevorzugt bestimmte Klebeeigenschaften, was ebenfalls zur Vergrößerung der Verweildauer beiträgt.

Um auch über einen längeren Zeitraum innerhalb des Körpers autark arbeiten zu können, kann die Vorrichtung außenseitig Elektroden zur galvanischen Stromerzeugung bei Kontakt mit Körperflüssigkeit aufweisen.
Das Mitführen einer Batterie zur Stromversorgung ist somit nicht erforderlich, so daß entsprechend Platz gespart werden kann oder für andere Einrichtungen zur Verfügung steht. Obgleich die batterielose Ausführung bevorzugt ist, kann in besonderen Fällen eine solche vorgesehen sein, gegebenenfalls auch als Pufferbatterie in Verbindung mit Elektroden zur Stromerzeugung bei Kontakt mit Körperflüssigkeit oder einer Ladespule.

Zur Überwachung des Behandlungsbereiches ist zumindest ein Sensor zur Bestimmung der Ansäuerung oder eines anderen Parameters im Behandlungsbereich vorgesehen, da beispielsweise Änderungen des pH-Wertes Rückschlüsse auf die metabolischen Aktivitäten der Tumor-Zellen zulassen und dadurch entsprechende Behandlungsanpassungen vorgenommen werden können. Dem liegt die Erkenntnis zugrunde, daß Wachstum und Ausbreitung von Tumoren als ein Prozeß zellulärer Selbst-Organisation betrachtet werden müssen, der, abgesehen von Veränderungen im zellulären Signal-Verarbeitungs-Apparat, wesentlich von der Mikro-Umgebung des Tumors gesteuert wird. Dabei kann der pH-Wert der Mikroumgebung des Tumors eine zentrale Schlüsselrolle spielen.
Wird beispielsweise ein pH-Sollwert von 7,4 vorgegeben, so erfolgt durch die Steuereinrichtung aufgrund der Messung des vorhandenen pH-Wertes als Istwert, eine geregelte Dosierung des medizinischen Wirkstoffes, bis der Sollwert, im Beispiel pH 7,4, erreicht ist. Der medizinische Wirkstoff kann ein Wirkstoff zur Neutralisation des pH-Gradienten sein. Weiterhin kommt ein Wirkstoff (Antagonist) zur Blockade der Protonenpumpe an den Zellmembranen der Tumorzellen oder ein Wirkstoff zur Blockade der molekularbiologischen Agentien (beispielsweise Antisens-Produkte) in Betracht. In Kombination mit der chemischen Beeinflussung des tumorösen Gewebes, kann auch eine physikalische Beeinflussung vorgenommen werden. Dies kann durch elektrische und/oder elektromagnetische Felder über Elektroden an der Vorrichtung mittels Iontophorese erfolgen. An die Elektroden kann eine Gleichspannung oder eine Wechselspannung angelegt werden.
Auch hierbei wird die Änderung des Feldes in Abhängigkeit von dem jeweiligen Meßwert vorgenommen, so daß auch diesbezüglich ein Regelkreis und somit eine gezielte Behandlung mit "Feedback"vorhanden ist.

Als Sensor zur Bestimmung der Ansäuerung der unmittelbaren Umgebung der Tumorzellen kann ein pH-Sensor auf Halbleiterbasis oder ein pH-Sensor auf der Basis einer Leitfähigkeits- und Impedanzmessung vorgesehen sein, wobei bei einem pH-Sensor auf Halbleiterbasis für diesen vorzugsweise wenigstens ein ionenselektiver Feldeffekttransistor (IS-FET) vorgesehen ist.
Mit einem pH-Sensor auf Halbleiterbasis ist eine hohe Meßgenauigkeit erzielbar und ein Sensor (gegebenenfalls Immunsensor) auf der Basis einer Leitfähigkeits- und Impedanzmessung läßt sich problemspezifisch in bestimmten Anwendungen (Leber, Magen) einfacher anwenden.
Gegebenenfalls ist zusätzlich zu wenigstens einem pH-Sensor wenigstens ein weiterer Sensor, insbesondere ein Ionen- oder Molekularsensor vorgesehen.
Mit diesen zusätzlichen Sensoren lassen sich außer pH-Wert-änderungen auch noch zusätzliche, therapierelevante Änderungen in der Mikroumgebung eines Tumors erfassen und aus diesen zusätzlichen Meßdaten können entsprechende Maßnahmen beim Applizieren des medizinischen Wirkstoffes abgeleitet werden.

Zweckmäßigerweise weist die Wirkstoffabgabeeinrichtung vorzugsweise wenigstens eine poröse Membran und eine Wirkstoff-Zuführung zu dieser Membrane auf, wobei sich bei der Wirkstoff-Zuführung eine Dosiereinrichtung befindet, die an eine Dosiersteuerung angeschlossen ist. Die Membran ist gegebenenfalls chemisch somodifizierbar, daß sie auf der zu behandelnden Stelle bevorzugt anheftet.
Dabei bilden der oder die Sensoren sowie die gegebenenfalls vorgesehene, poröse Membrane eine Auflage- und Kontaktfläche für den zu behandelnden Gewebebereich.
Bei dieser Auflage- und Kontaktfläche für den zu behandelnden Gewebebereich können auch wenigstens zwei Elektroden zu iontophoretischen Zwecken vorgesehen sind, die über elektrische Leitungen mit einer Spannungsquelle verbunden sind.
Damit ist eine regional begrenzte und dosierte Zuführung von Wirkstoff möglich.

Eine Ausführungsform der Erfindung sieht vor, daß die Funktionseinheit wenigstens einen Wirkstoff-Vorratsbehälter, eine oder mehrere, mit der porösen Membrane oder dergleichen verbundene Dosiereinrichtung(en) mit Dosiersteuerung sowie zumindest einen pH-Sensor aufweist.
Für eine physikalische Beeinflussung besteht auch die Möglichkeit, daß die Funktionseinheit wenigstens einen pH-Sensor, wenigstens zwei Elektroden zu iontophoretischen Zwecken, eine Spannungsquelle sowie eine Steuereinrichtung aufweist. Bedarfsweise kann die Funktionseinheit sowohl Einrichtungen für eine chemische als auch für eine physikalische Behandlung beziehungsweise Beeinflussung aufweisen. Eine solche Vorrichtung kann als komplette, funktionstüchtige Einheit innerhalb des Verdauungstraktes über einen vorgesehenen Behandlungszeitraum verbleiben.

Zusätzliche Ausgestaltungen der Erfindung sind in den weiteren Unteransprüchen aufgeführt.
Nachstehend ist die Erfindung mit ihren wesentlichen Einzelheiten anhand der Zeichnungen noch näher erläutert.
Es zeigt:
- Fig.1 bis 3: schematisierte Darstellungen einer Vorrichtung in unterschiedlichen Ausführungsformen mit jeweils drei sandwichartig übereinander angeordneten Baugruppen,
- Fig. 4: eine schematisierte Seitenansicht einer anderen Ausführungsform und
- Fig. 5: eine schematisierte Seitenansicht einer wiederum abgewandelten Ausführungsform.

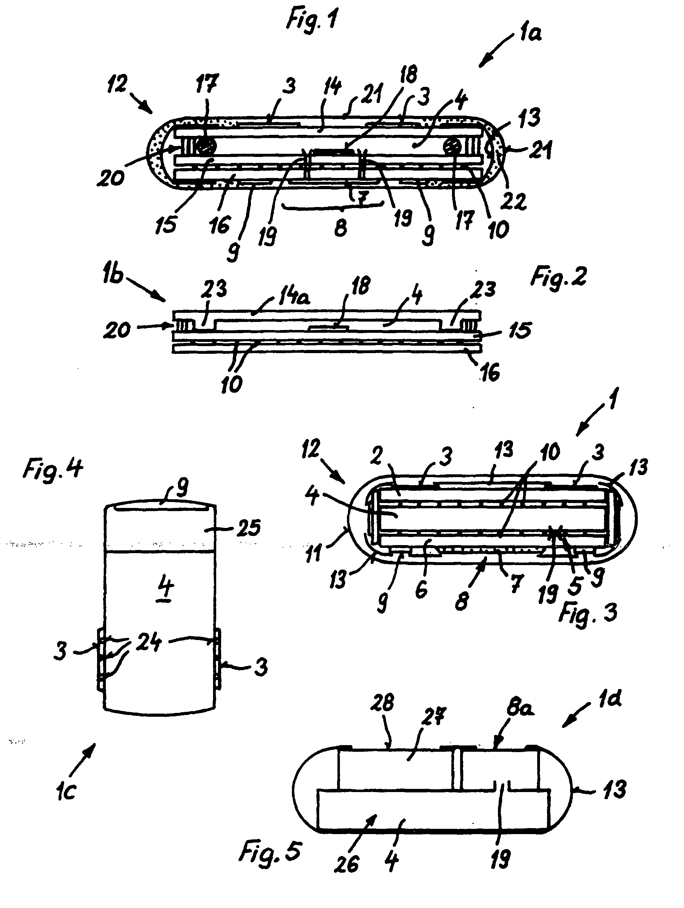

Eine in Figur 3 gezeigte Vorrichtung 1 dient zum Applizieren von medizinischem Wirkstoff im Bereich von lebendem Zellgewebe. Es kann sich dabei insbesondere um einen zu behandelnden Tumor handeln, der sich innerhalb des Verdauungstraktes befindet. Die Vorrichtung 1 ist als schluckbare Kapsel ausgebildet und weist dementsprechende kleine Abmessungen mit einer Länge von beispielsweise 20 mm und einem Durchmesser beziehungsweise einer Dicke von einigen Millimetern, beispielsweise 5 mm auf.
Die mechanische Absetzung und Positionierung der Vorrichtung kann sowohl im Magen- als auch im Darmbereich gegebenenfalls durch eine endoskopische Technik unterstützt werden.
Wie gut in Figur 3 erkennbar, setzt sich die Vorrichtung 1 im wesentlichen aus drei sandwichartig übereinander angeordneten und miteinander verbundenen Baugruppen zusammen.
An einer Außenseite befindet sich dabei ein Steuerchip 2, der eine Steuereinrichtung beinhaltet, mittels der die chemische und/oder physikalische Behandlung in Abhängigkeit von zugeführten Meßdaten gesteuert wird.
An der Außenseite dieses Steuerchips befinden sich Elektroden 3, die zur galvanischen Stromerzeugung bei Kontakt mit Körperflüssigkeit dienen.

Mit dem Steuerchip 2 ist ein Behälter 4 zur Aufnahme eines Wirkstoffes verbunden, der über einen Verbindungskanal 5 mit einer Wirkstoffabgabeeinrichtung 6 verbunden ist. Diese Einrichtung 6 weist ein vorzugsweise durch eine poröse Membrane 7 gebildetes Abgabefenster 8 auf. Benachbart zu dem Abgabefenster 8 befinden sich Sensoren 9, von denen wenigstens einer ein pH-Sensor ist, mittels dem der pH-Wert des zu behandelnden Bereiches überwacht werden kann.
Die Baugruppen 2, 4 und 6 können als vorzugsweise etwa gleichflächige Bauteile ausgebildet sein, die an ihren zueinander gewandten Seiten Kontaktpads 10 aufweisen, mittels denen die Baugruppen vorzugsweise mittels Flip-Chip-Technik miteinander verbindbar sind.
Die aus den einzelnen Baugruppen bestehende Einheit weist eine Umhüllung 11 auf, die zeit- und/oder stoffabhängig von der Körperflüssigkeit auflösbar ist. Die stoffliche Zusammensetzung dieser Umhüllung 11 ist in Abhängigkeit davon vorgesehen, wo die Vorrichtung 1 innerhalb des Verdauungstraktes aktiviert werden soll. Dabei ist insbesondere auch eine Abstimmung auf die innerhalb des Verdauungstraktes einwirkenden, unterschiedlichen Körperflüssigkeiten vorgesehen.
Es kann auch eine vom Stoff her unterschiedliche Umhüllung vorgesehen sein, die im Bereich der Elektroden 3 anders zusammengesetzt ist, als im gegenüberliegenden Bereich, wo sich das Abgabefenster und die Sensoren befinden. Beispielsweise kann es vorgesehen sein, daß nach dem Schlucken der Vorrichtung 1 zunächst die Umhüllung 11 bei den Elektroden 3 zur Stromerzeugung aufgelöst wird, so daß dann die Vorrichtung funktionsbereit ist. Ist dann noch die Umhüllung im Bereich des Abgabefensters 8 und der Sensoren 9 aufgelöst, beginnt die Behandlung zum Beispiel durch gezielte Abgabe von Wirkstoff bei dem Abgabefenster 8.
Außer der Umhüllung 11 weist die Kapsel 12 eine innere Schutzumhüllung 13 auf, die im Bereich der nach außen wirkenden Teile - Elektroden, Sensoren, Abgabefenster - entsprechende Unterbrechungen aufweist.

Fig. 1 zeigt eine weitere Ausführungsform einer Vorrichtung 1a, die ebenfalls als Kapsel 12 ausgebildet ist. Auch bei dieser Ausführungsform ist ein dreischichtiger Aufbau mit Hilfe von drei Chips 14, 15, 16 vorgesehen. Zwischen dem außen angeordneten Chip 14 und dem dazu benachbarten Chip 15 ist zur Bildung des Wirkstoff-Vorratsbehälters 4 ein entsprechend bemessener Abstand vorgesehen und als Abstandhalter dient ein umlaufender Dichtring 17 zwischen diesen beiden Chips 14 und 15.
Innerhalb des kavernenartigen Behälters 4 ist wenigstens ein Heizwiderstand 18 zum Erwärmen des im Behälter 4 befindlichen Wirkstoffes vorgesehen. Der Behälter 4 weist im Ausführungsbeispiel neben dem Heizwiderstand 18 zwei Kapillarkanäle 19 mit sich anschließender Verbindung zu dem insbesondere durch eine poröse Membrane 7 gebildeten Abgabefenster 8 auf. Beim Erwärmen der im Behälter 4 befindlichen Wirkstofflüssigkeit durch den Heizwiderstand 18 dehnt sich die Flüssigkeit aus und wird über die Kapillarkanäle 19 zur Membrane 7 abgegeben.
Der Dichtring 17 ist zum Außenrand der Chips 14 und 15 beabstandet angeordnet, so daß außenseitig noch genügend Platz verbleibt, um über Verbindungskontaktierungen 20, beispielsweise eine Drahtbondung, eine elektrische Verbindung zwischen den beiden Chips 14,15 herstellen zu können. Die Chips 15 und 16 weisen Kontaktpads 10 auf und sind insbesondere mittels Flip-Chip-Technik miteinander verbunden.
Die in Fig. 1 gezeigte Kapsel 12a weist eine Umhüllung 21 mit einem punktiert angedeuteten Füllmaterial oder Aufschäumsystem 21 insbesondere auf enzymatischer Basis auf.
Diese Umhüllung mit oder aus entsprechendem Material reagiert immunbiochemisch oder immunenzymatisch bei Erreichen eines Entzündungsortes oder eines tumorösen Ortes. Die Kapsel 12a kann dadurch beim Behandlungsort festgelegt oder zumindest ihre Verweildauer dort vergrößert werden. Durch das Aufschäumen mit entsprechender Volumenvergrößerung, wobei auch ein zeitweises Festkleben in dem Behandlungsbereich erfolgen kann, wird der Weitertransport der Kapsel 12a innerhalb des Verdauungstraktes zumindest behindert, so daß eine ausreichend lange Behandlungszeit zur Verfügung steht.

Fig. 2 zeigt eine prinzipiell mit Fig. 1 vergleichbar aufgebaute Vorrichtung 1b, die ebenfalls drei übereinander geschichtete Chips 14a, 15 und 16 aufweist. Der Chip 14a weist hierbei an seiner dem Chip 15 zugewandten Seite eine insbesondere durch Ätzung gebildete Ringwand 23 als Abstandhalter und zur Bildung des Wirkstoff-Vorratsbehälters 4 zwischen diesen beiden Chips 14a und 15 auf. Die Stirnseite dieser Ringwand ist mit dem daran anliegenden Chip 15 dicht verbunden, was vorzugsweise durch eutektisches Bonden erreicht wird. Im übrigen kann der Aufbau der Vorrichtung 1b dem der in Fig. 1 gezeigten Vorrichtung 1a entsprechen.

Fig.4 zeigt noch eine abgewandelte Ausführungsform einer Vorrichtung 1c, die ebenfalls kapselförmig ausgebildet ist. Die Anordnung der einzelnen Baugruppen weicht hier jedoch von den anderen Ausführungsbeispielen ab. Die Vorrichtung 1c weist ebenfalls einen Wirkstoff-Vorratsbehälter 4 auf, der Abgabeöffnungen 24 im Bereich von Referenz-Elektroden 3 hat. An einem Kopfende der Kapsel befindet sich ein Elektronikteil mit der Steuereinrichtung für die physikalische und/oder chemische Behandlung und die Verarbeitung der von dem oder den Sensoren kommenden Meßwerte. Der oder die Sensoren 9 befinden sich an einem stirnseitigen Außenende der Kapsel beim Elektronikteil 25.
Eine wiederum abgewandelte Ausführungsform zeigt Fig. 5. Mit 26 ist dabei ein vorzugsweise als Chip ausgebildetes Trägerteil bezeichnet, in dem sich der Wirkstoff-Vorratsbehälter 4 befindet. Bei einer Ausführungsform als Chip kann in diesen eine Kaverne als Vorratsbehälter 4 sowie auch ein Kapillarkanal 19 eingeätzt sein. Der Kapillarkanal 19 führt zu einem Abgabefenster 8a, über das der Wirkstoff nach außen abgegeben wird. Das Abgabefenster 8a mit Steuerventil und Dosiersteuerung kann als separater Chip ausgebildet sein.
Auch der dritte Funktionsblock 27 ist vorzugsweise als Chip ausgebildet und beinhaltet die Steuerelektronik und an seiner Außenseite 28 Sensoren und gegebenenfalls auch Elektroden für die Stromerzeugung.
13 ist eine Schutzumhüllung, die im Bereich des Abgabefensters 8a und der Außenseite 28 ausgespart ist.
Wie in Fig. 4 und 5 angedeutet, können die als schluckbare Kapseln ausgebildeten Vorrichtungen 1c und 1d ohne eine unter dem Einfluß von Körperflüssigkeit sich auflösende Umhüllung 21 (vgl. Fig. 1 und 3) verwendet werden. Die Aktivierung der Wirkstoffabgabeeinrichtung und/oder der Elektroden erfolgt hierbei in Abhängigkeit von laufend gemessenen Auslöse-parametern, beispielsweise des pH-Wertes. Auch kann eine Messung auf enzymatischer Basis erfolgen. Sind entsprechende Auslöse-Parameter vorhanden, so wird die Aktivierung der Wirkstoffabgabe und/oder der Elektroden veranlaßt.

## Patentansprüche

1. Vorrichtung (1,1a,1b,1c,1d) zum Behandeln von malignen, tumorösen Gewebebereichen, mit wenigstens einem Meßsensor zur Bestimmung chemischer oder physikalischer Signalmuster in der unmittelbaren Umgebung des tumorösen Gewebebereiches, mit einer Steuereinrichtung sowie mit zumindest einer Behandlungseinrichtung, die wenigstens einen Wirkstoff-Vorratsbehälter (4), eine Dosiereinrichtung mit Dosiersteuerung und eine Wirkstoffabgabeeinrichtung (6) zur chemischen Beeinflussung und optional Elektroden zur physikalischen Beeinflussung des zu behandelnden, tumorösen Gewebebereiches aufweist und wobei der oder die Sensoren (9), die Wirkstoffabgabeeinrichtung (6) sowie die Behandlungs-Elektroden (3) mit der Steuereinrichtung für eine in Abhängigkeit von den Meßwerten der Tumorzellen gesteuerte, physikalische und/oder chemische Behandlung des tumorösen Gewebebereiches mit laufender Dosiernachführung mit dem zu applizierenden Wirkstoff verbunden sind, **dadurch gekennzeichnet, daß** die Vorrichtung kapselförmig oder drageeförmig zum Schlucken ausgebildet ist und eine Umhüllung (11) aufweist, die zumindest den oder die Sensoren (9) abdeckt, daß die Umhüllung unter dem Einfluß von Körperflüssigkeit zumindest bereichsweise auflösbar ist, daß ein Sensor zum Erfassen von Auslöse-Parametern und die Steuereinrichtung mittels eines Schwellwertschalters zur Aktivierung der Wirkstoffabgabeeinrichtung und/oder der Elektroden bei Überschreitung eines vorgegebenen oder vorgebbaren Sollwertes ausgebildet sind und daß zur Wirkstoffabgabe innerhalb des Wirkstoff-Vorratsbehälters (4) wenigstens ein Heiz-Widerstand (18) zum Erwärmen und zur Abgabe von Wirkstoff vorgesehen ist.

2. Vorrichtung nach Anspruch 1, **dadurch gekennzeichnet, daß** zumindest zur Vergrößerung der Verweildauer der Kapsel oder des Dragees am Behandlungsort, diese eine Umhüllung (21) miteinem Füllmaterial oder Aufschäumsystem (22) insbesondere auf enzymatischer Basis aufweist, die für eine immunbiochemische und/oder immunenzymatische Reaktion bei Erreichen eines Entzündungsortes oder eines tumorösen Ortes ausgebildet ist.

3. Vorrichtung nach Anspruch 2 oder 3, **dadurch gekennzeichnet, daß** außer dem oder den Sensoren (9), die Abgabestelle der Wirkstoffabgabeeinrichtung (6), sowie gegebenenfalls die Behandlungs-Elektroden im Ausgangszustand von der Umhüllung (11) abgedeckt sind.

4. Vorrichtung nach einem der Ansprüche 1 bis 3, **dadurch gekennzeichnet, daß** die Umhüllung (11) der Kapsel oder des Dragees zeit- und/oder stoffabhängig von der Körperflüssigkeit auflösbar ist.

5. Vorrichtung nach einem der Ansprüche 1 bis 4, **dadurch gekennzeichnet, daß** sie außenseitig Elektroden (3) zur galvanischen Stromerzeugung bei Kontakt mit Körperflüssigkeit aufweist.

6. Vorrichtung nach einem der Ansprüche 1 bis 5, **dadurch gekennzeichnet, daß** die zur Funktion erforderlichen Einrichtungen, insbesondere die Steuereinrichtung, die Behandlungseinrichtung, die Wirkstoffabgabeeinrichtung und dergleichen Baugruppen, sandwichartig miteinander verbunden sind und eine Baueinheit, insbesondere eine komplette Funktionseinheit bilden.

7. Vorrichtung nach einem der Ansprüche 1 bis 6, **dadurch gekennzeichnet, daß** die einzelnen Baugruppen im wesentlichen als Chips ausgebildet sind und insbesondere in Flip-Chip-Technik miteinander verbunden sind.

8. Vorrichtung nach einem der Ansprüche 1 bis 7, **dadurch gekennzeichnet, daß** die Wirkstoffabgabeeinrichtung vorzugsweise wenigstens eine poröse, strukturierte Membrane (7) und eine Wirkstoff-Zuführung zu dieser Membrane aufweist und daß sich bei der Wirkstoff-Zuführung eine Dosiereinrichtung befindet, die an eine Dosiersteuerung angeschlossen ist.

9. Vorrichtung nach einem der Ansprüche 1 bis 8, **dadurch gekennzeichnet, daß** die Funktionseinheit wenigstens einen pH- Sensor (9) sowie bedarfsweise wenigstens zwei Elektroden zu iontophoretischen Zwecken, eine Spannungsquelle sowie eine Steuereinrichtung aufweist.

10. Vorrichtung nach einem der Ansprüche 1 bis 9, **dadurch gekennzeichnet, daß** der oder die Sensoren (9) sowie die Membrane eine Auflage- und Kontaktfläche für den zu behandelnden Gewebebereich bilden.

11. Vorrichtung nach einem der Ansprüche 1 bis 10, **dadurch gekennzeichnet, daß** der oder die Sensoren (9) benachbart zu der Membrane (7) angeordnet sind.

12. Vorrichtung nach einem der Ansprüche 1 bis 11, **dadurch gekennzeichnet, daß** bei der Auflage- und Kontaktfläche für den zu behandelnden Gewebebereich wenigstens zwei Elektroden zu iontophoretischen Zwecken vorgesehen sind.

13. Vorrichtung nach einem der Ansprüche 1 bis 12, **dadurch gekennzeichnet, daß** der Sensor zur Bestimmung der Ansäuerung der unmittelbaren Umgebung der Tumorzellen ein pH-Sensor auf Halbleiterbasis oder ein pH-Sensor auf der Basis einer Leitfähigkeits- und Impedanzmessung ist.

14. Vorrichtung nach einem der Ansprüche 9 bis 13, **dadurch gekennzeichnet, daß** als pH-Sensor (9) wenigstens ein ionenselektiver Feldeffekttransistor (IS-FET) vorgesehen ist.

15. Vorrichtung nach einem der Ansprüche 9 bis 14, **dadurch gekennzeichnet, daß** zusätzlich zu wenigstens einem pH-Sensor wenigstens ein weiterer Sensor, insbesondere ein Ionen-oder Molekularsensor vorgesehen ist.

16. Vorrichtung nach einem der Ansprüche 1 bis 15, **dadurch gekennzeichnet, daß** innerhalb der Auflage- und Kontaktfläche für den zu behandelnden Gewebebereich, gegebenenfalls am Umfangsbereich der porösen Membrane (7), mehrere, vorzugsweise zueinander beabstandete Sensoren (9) angeordnet sind.

17. Vorrichtung nach einem der Ansprüche 1 bis 16, **dadurch gekennzeichnet, daß** zur Bildung des Wirkstoff-Vorratsbehälters (4) insbesondere zwischen einem äußeren Chip (14) und einem benachbarten, insbesondere den oder die HeizWiderstände aufweisenden Chip (15), ein den Behälterraum umgrenzender, abdichtender Abstandhalter (17,23) vorgesehen ist.

18. Vorrichtung nach Anspruch 17, **dadurch gekennzeichnet, daß** als Abstandhalter ein Dichtring (17), insbesondere ein O-Ring vorgesehen ist.

19. Vorrichtung nach Anspruch 17, **dadurch gekennzeichnet, daß** einer der beiden benachbarten Chips an der dem anderen Chip zugewandten Seite eine insbesondere durch Ätzung gebildete Ringwand (23) aufweist, und daß deren Stirnseite zur dichten Verbindung mit dem anderen Chip vorzugsweise mit diesem eutektisch gebondet ist.

20. Vorrichtung nach einem der Ansprüche 17 bis 19, **dadurch gekennzeichnet, daß** der Abstandhalter (17,23) zwischen den beiden benachbarten Chips zum Außenrand beabstandet ist und daß in diesem Außenrandbereich außenseitig des Abstandhalters Verbindungskontaktierungen (20) zwischen den beiden Chips angeordnet sind.

21. Vorrichtung nach einem der Ansprüche 1 bis 20, **dadurch gekennzeichnet, daß** der Wirkstoff-Vorratsbehälter (4) wenigstens einen Kapillarkanal (19) zur Wirkstoffabgabe aufweist.

## Claims

1. A device (1, 1a, 1b, 1c, 1d) for the treatment of malignant, tumorous areas of tissue, having at least one sensor for determining chemical or physical signal patterns in the direct vicinity of the tumourous area of tissue, having a control device and also having at least one treatment device, which comprises at least one drug reservoir (4), a dosing device with a dosing control device and a drug dispensing device (6) for chemically influencing and optionally electrodes for physically influencing the tumorous area of tissue to be treated, and wherein the sensor or sensors (9), the drug dispensing device (6) and also the treatment electrodes (3) are connected to the control device for a physical and/or chemical treatment of the tumorous area of tissue that is controlled in dependence on the measured values of the tumour cells with ongoing dosing tracking with the drug to be applied,
**characterised in that** the device is constructed in the form of a capsule or coated tablet for swallowing and comprises a coating (11), which covers at least the sensor or sensors (9),
**in that** the coating is soluble at least in some areas under the effect of body fluid,
**in that** a sensor is provided to detect triggering parameters and the control device is constructed by means of a threshold value switch to active the drug dispensing device and/or the electrodes when a predetermined or predeterminable set value is exceeded
and **in that** at least one heating resistor (18) is provided to head and discharge a drug for the dispensing a drug inside the drug reservoir (4).

2. A device according to Claim 1,
**characterised in that** at least to increase the retention time of the capsule or the coated tablet at the treatment site, said capsule or coated tablet comprises a coating (21) having a filling material or foaming system (22), in particular on an enzymatic base, which is designed for an immunobiochemical and/or immunoenzymatic reaction when an inflammation site or a tumorous site is reached.

3. A device according to Claim 2 or 3,
**characterised in that** apart from the sensor or sensors (9), the dispensing site of the drug dispensing device (6) and, where appropriate, the treatment electrodes are covered by the coating (11) in the initial state.

4. A device according to one of Claims 1 to 3,
**characterised in that** the coating (11) of the capsule or the coated tablet is soluble in time-dependent manner and/or depending on the substances in the body fluid.

5. A device according to one of Claims 1 to 4,
**characterised in that** it comprises electrodes (2) on the outside for galvanic electricity generation upon contact with body fluid.

6. A device according to one of Claims 1 to 5,
**characterised in that** the devices required for the function, in particular the control device, the treatment device, the drug dispensing device and similar modules are connected to one another in the manner of a sandwich and form a structural unit, in particular a complete functional unit.

7. A device according to one of Claims 1 to 6,
**characterised in that** the individual modules are essentially constructed as chips and are connected to each other in particular using flip-chip technology.

8. A device according to one of Claims 1 to 7,
**characterised in that** the drug dispensing device preferably comprises at least one porous, structured membrane (7) and a drug supply to this membrane
and **in that** a dosing device which is connected to the dosing control device is situated near the drug supply.

9. A device according to one of Claims 1 to 8,
**characterised in that** the functional unit comprises at least one pH sensor (9) and if necessary at least two electrodes for ionotophoretic purposes, a voltage source and also a control device.

10. A device according to one of Claims 1 to 9,
**characterised in that** the sensor or sensors (9) and also the membrane form a support and contact surface for the tissue region to be treated.

11. A device according to one of Claims 1 to 10,
**characterised in that** the sensor or sensors (9) are disposed adjacent to the membrane (7).

12. A device according to one of Claims 1 to 11,
**characterised in that** at least two electrodes for ionotophoretic purposes are provided near the support and contact surface for the tissue area to be treated.

13. A device according to one of Claims 1 to 12,
**characterised in that** the sensor for determining the acidulation of the immediate vicinity of the tumour cells is a pH sensor on a semiconductor base or a pH sensor on the basis of a conductivity and impedance measurement.

14. A device according to one of Claims 9 to 13,
**characterised in that that** least one ion-selective field effect transistor (IS-FET) is provided as the pH sensor (9).

15. A device according to one of Claims 9 to 14,
**characterised in that** in addition to at least one pH sensor, at least one other sensor, in particular an ion or a molecular sensor, is provided.

16. A device according to one of Claims 1 to 15,
**characterised in that** several sensors (9), preferably spaced from each other, are disposed inside the support and contact surface for the tissue area to be treated, possibly on the peripheral region of the porous membrane (7).

17. A device according to one of Claims 1 to 16,
**characterised in that** a spacer (17, 23) that surrounds the reservoir? and has a sealing action is provided to form the drug reservoir (4) in particular between an outer chip (14) and an adjacent chip (15) that in particular comprises the heating resistor or resistors.

18. A device according to Claim 17,
**characterised in that** a sealing ring (17), in particular an O ring, is provided as the spacer.

19. A device according to Claim 17,
**characterised in that** one of the two adjacent chips comprises at the side facing the other chip an annular wall (23) formed in particular by etching,
and **in that** its end side is preferably eutectically bonded with the other chip for tight connection with said chip.

20. A device according to one of Claims 17 to 19,
**characterised in that** the spacer (17, 23) is spaced between the two adjacent chips at the outer edge
and **in that** in this outer edge region outside the spacer connection contacts (20) are disposed between the two chips.

21. A device according to one of Claims 1 to 20,
**characterised in that** the drug reservoir (4) comprises at least one capillary duct (19) for dispensing the drug.

## Revendications

1. Dispositif (1, 1a, 1b, 1c, 1d) pour le traitement de zones tissulaires tumorales malignes, présentant au moins un capteur de mesure pour déterminer les modèles de signaux chimiques ou physiques dans l'environnement immédiat de la zone tissulaire tumorale avec une installation de commande ainsi qu'avec au moins une installation de traitement qui présente au moins un réservoir de stockage de principe actif (4), un appareil de dosage avec une commande de dosage, un appareil de délivrance de principe actif (6) pour l'influence chimique des électrodes en option pour l'influence physique de la zone tissulaire tumorale à traiter, dans lequel le ou les capteurs (9), le dispositif de délivrance du principe actif (6) ainsi que les électrodes de traitement (3) sont reliés à l'installation de commande pour un traitement chimique et/ou physique de la zone tissulaire tumorale, commandé en fonction des valeurs mesurées des cellules cancéreuses en maintenant le dosage actuel du principe actif à appliquer,
**caractérisé en ce que**
le dispositif se présente sous la forme de capsule ou de dragée en vue de l'avaler et qu'il présente un enrobage (11) qui recouvre au moins le ou les capteurs (9), l'enrobage est soluble sous l'influence du liquide corporel au moins dans cette zone, un capteur est formé pour récupérer les paramètres de dissolution et l'installation de commande est formé pour activer au moyen d'un commutateur de valeurs seuil l'appareil de délivrance du principe actif et/ou les électrodes lors du dépassement du point de consigne prédéterminé ou pouvant être prédéterminé, et à l'intérieur du réservoir de stockage de principe actif (4) au moins une résistance chauffante (18) est prévue pour chauffer et délivrer le principe actif.

2. Dispositif selon la revendication 1,
**caractérisé en ce qu'**
au moins pour l'agrandissement de la durée de séjour de la capsule ou de la dragée sur le site de traitement, il présente un enrobage (21) avec une charge ou un système de moussage (22) notamment à base enzymatique, lequel est formé pour une réaction immunobiologique et/ou enzymatique une fois le site d'inflammation et/ou le site de la tumeur atteint.

3. Dispositif selon la revendication 2 ou 3,
**caractérisé en ce qu'**
outre le ou les capteurs (9), le site de délivrance du dispositif de délivrance du principe actif (6), ainsi le cas échéant que les électrodes de traitement sont recouverts à l'état de sortie de l'enrobage (11).

4. Dispositif selon l'une quelconque des revendications 1 à 3,
**caractérisé en ce que**
l'enrobage (11) de la capsule ou de la dragée peut être dissous en fonction du temps et/ou de la substance par le liquide corporel.

5. Dispositif selon l'une quelconque des revendications 1 à 4,
**caractérisé en qu'**
il présente des électrodes (3) du côté externe pour la production de courant galvanique lors du contact avec le liquide corporel.

6. Dispositif selon l'une quelconque des revendications 1 à 5,
**caractérisé en ce que**
les appareils requis pour le fonctionnement, notamment l'installation de commande, l'installation de traitement, l'appareil de délivrance du principe actif et les modules similaires, sont reliés les uns aux autres sous forme de sandwich et forment un module notamment une unité de fonctionnement complète.

7. Dispositif selon l'une quelconque des revendications 1 à 6,
**caractérisé en ce que**
les différents modules sont formés majoritairement sous la forme d'une puce, et sont notamment reliés les uns aux autres dans une technique Flip-Chip.

8. Dispositif selon l'une quelconque des revendications 1 à 7,
**caractérisé en ce que**
l'appareil de délivrance du principe actif présente, de préférence, au moins une membrane structurée poreuse (7) et une alimentation en principe actif pour cette membrane, et lors de l'alimentation en principe actif, un appareil de dosage qui s'y trouve, lequel est raccordé à une commande de dosage.

9. Dispositif selon l'une quelconque des revendications 1 à 8,
**caractérisé en ce que**
l'unité de fonctionnement présente au moins un capteur de pH (9) et, au besoin, au moins deux électrodes à des fins ionophorétiques, une source de tension ou un appareil de commande.

10. Dispositif selon l'une quelconque des revendications 1 à 9,
**caractérisé en ce que**
le ou les capteurs (9) ainsi que la membrane forment une surface de contact et de dépôt pour la zone tissulaire à traiter.

11. Dispositif selon l'une quelconque des revendications 1 à 10,
**caractérisé en ce que**
le ou les capteurs (9) sont à proximité de la membrane (7).

12. Dispositif selon l'une quelconque des revendications 1 à 11,
**caractérisé en ce que**
pour la surface de contact et de dépôt, au moins deux électrodes à des fins ionophorétiques sont prévues pour la zone tissulaire à traiter.

13. Dispositif selon l'une quelconque des revendications 1 à 12,
**caractérisé en ce que**
le capteur permettant de déterminer l'acidité de l'environnement immédiat des cellules cancéreuses est un capteur de pH sur la base d'un semi-conducteur ou un capteur de pH sur la base d'une mesure d'impédance ou de conductance.

14. Dispositif selon l'une quelconque des revendications 9 à 13,
**caractérisé en ce que**
le capteur de pH (9) est au moins un transistor à effet de champ ioniquement sélectif (IS-FET) est prévu.

15. Dispositif selon l'une des revendications 9 à 14,
**caractérisé en ce qu'**
en plus d'au moins un capteur de pH, au moins un autre capteur notamment un capteur moléculaire ou ionique est prévu.

16. Dispositif selon l'une quelconque des revendications 1 à 15,
**caractérisé en ce que**
plusieurs capteurs, de préférence distants les uns des autres (9), sont prévus à l'intérieur de la zone de contact ou de dépôt pour la zone tissulaire à traiter, le cas échéant sur la zone périphérique de la membrane poreuse (7).

17. Dispositif selon l'une quelconque des revendications 1 à 16,
**caractérisé en ce que**
pour la formation du réservoir de stockage de principe actif (4) un espaceur étanche (17, 23) délimitant l'espace du réservoir est prévu notamment entre une puce plus externe (14) et une puce (15) voisine qui, notamment comporte la ou les résistances chauffante.

18. Dispositif selon la revendication 17,
**caractérisé en ce que**
l'espaceur est un joint d'étanchéité (17), notamment un joint torique.

19. Dispositif selon la revendication 17,
**caractérisé en ce qu'**
une des deux puces voisines présente une paroi circulaire formée notamment par attaque chimique (23) sur le côté dirigé vers l'autre puce, et son côté frontal est lié de façon eutectique en vue de former une liaison étanche avec l'autre puce de préférence avec celui-ci.

20. Dispositif selon l'une quelconque des revendications 17 à 19,
**caractérisé en ce que**
l'espaceur (17, 23) entre les deux puces voisines est distant du pourtour et des contacts de liaison (20) entre les deux puces sont agencés dans cette zone périphérique, côté externe du séparateur.

21. Dispositif selon l'une quelconque des revendications 1 à 20,
**caractérisé en ce que**
le réservoir de stockage de principe actif (4) présente au moins un canal capillaire (19) pour la délivrance du principe actif.
